# EUROPEAN PATENT SPECIFICATION

(11) **EP 3 107 602 B1**
(45) Date of publication and mention of the grant of the patent: **28.03.2018**
(21) Application number: 14704829.2
(22) Date of filing: 18.02.2014
(51) Int. Cl.: A61M 5/28

(54) **DELIVERY SYSTEM FOR DELIVERING MEDICAL OR PHARMACEUTICAL COMPOUNDS**
AUSGABESYSTEM ZUR AUSGABE VON MEDIZINISCHEN ODER PHARMAZEUTISCHEN VERBINDUNGEN
SYSTÈME D'ADMINISTRATION POUR ADMINISTRER DES COMPOSÉS MÉDICAUX OU PHARMACEUTIQUES

(43) Date of publication of application: 28.12.2016
(73) Proprietor: Skufca, Peter, 78333 Stockach (DE)
(72) Inventor: Skufca, Peter, 78333 Stockach (DE)
(74) Representative: Winter, Brandl, Fürniss, Hübner, Röss, Kaiser, Polte - Partnerschaft mbB
(86) International application number: PCT/EP2014/053146
(87) International publication number: WO 2015/124173

(56) References cited:
- EP-A1- 2 535 073

## Description

The present invention refers to a delivery system for delivering medical or pharmaceutical compounds.

A prior art delivery system for delivering medical or pharmaceutical compounds is described in EP 2 535 073 A1 and comprises a first container storing the compounds, a closure element accommodated within the first container, and an extraction unit adapted to extract the compounds automatically, once the closure element has been manually penetrated. In the known system, the first container is accommodated within a second container which serves, on the one hand, as holding means for holding an energy unit used to carry out the above mentioned automatic function and, on the other hand, as support means to assist the first container in maintaining the extraction unit in a well defined positional relationship with respect to the first container. That is, in order to put into effect the above mentioned automatic delivery function, a container-container-arrangement is provided that renders the overall structure complicate and accident-sensitive.

It is an object of the present invention to improve the reliability and easy applicability of the above known system by providing a delivery system for delivering medical or pharmaceutical compounds that dispenses with the above mentioned automatic function.

This object is solved by a delivery system having the features of claim 1. Advantageous embodiments and variations are defined in the dependent claims.

A delivery system for delivering medical or pharmaceutical compounds according to the present invention comprises a (a) container unit, comprising (a-a) a container for storing the compounds, wherein the container has a circumferential wall, a first end closed by a container bottom and an open second end, and wherein the container unit has a longitudinal axis, and (a-b) a closure element accommodated within the container in a fluid-tight contact with the circumferential wall, and (b) an extraction unit comprising a penetration and discharge means having a hollow needle adapted to penetrate the closure element. The extraction unit is fitted to the container so as to be movable relatively to the container along the longitudinal axis towards the container bottom. The extraction unit, preferably the penetration and discharge means, comprises a first link motion portion, and the container comprises a second link motion portion, and one of the first link motion portion and the second link motion portion is provided with a projection and the respective other one is provided with a guiding groove, the projection and the guiding groove being engageable with each other to form a link motion adapted to make the extraction unit move relatively to the container in a predetermined way towards the container bottom thereby to make the hollow needle penetrate the closure element. As the penetration and discharge means having the hollow needle is moved with the extraction unit relatively to the container, the motion link provides guidance of the hollow needle towards and into the closure element thereby to make the hollow needle penetrate the closure element.

The word stems deliver, extract, and discharge each emphasize a specific perspective. That is, deliver refers to the inventive system in its entirety in the general meaning of dispense or give off or the more specific meaning of administer in case the subject the compounds are delivered to is an individual, extract refers to the extraction unit in order to focus on the process or operation of removing or taking out the compounds disregarding the purpose of this process, i.e. irrespective of what is aimed to be achieved with the compounds, and discharge refers to the transport of the compounds from the container through the hollow needle to the outside of the (entire) system, i.e. objectively to the penetration and discharge means.

The medical or pharmaceutical compounds are liquids that are in contact with the container and the closure element. Therefore, the container together with the closure element may form or advantageously form a primary package for the compounds conform with the Guidelines on packaging for pharmaceutical Products, issued in the WHO Technical Report Series, No. 902, 2002, determining or defining that a primary packaging must protect the pharmaceutical or medical products against all adverse external influences that may affect its quality or potency such as, for example, light, moisture, oxygen, biological contamination or mechanical damage. In particular, such a primary packaging must not interact physically or chemically with the contended medical or pharmaceutical compounds in any way that would alter their quality. Specifically, a primary packaging must protect the contents from extraneous matter, from loss of the substance, and from efflorescence, deliquescence or evaporation under normal conditions of handling, shipment or storage.

As above mentioned, the container unit comprises the container and the closure element. That is, the container unit comprises at least these elements but may contain more than these elements.

Advantageously, the container is essentially or overall of a right-cylindrical shape, i.e. of the shape of a mathematical cylinder having its axis (the above mentioned longitudinal axis) perpendicular to its base. That is, the inventive container may be thought of as being made up of a tube or barrel (its circumferential or side wall) of undefined cross-section, having a longitudinal axis perpendicular to each of virtual planes closing its ends, where one of these ends (the first end) is, preferably integrally, connected with or firmly closed (i.e. not non-destructively removable) by the container bottom that may or may not completely lie within the respective virtual plane (it may, for example, be curved or bulged). Preferably, the cross-section is symmetric with respect to the longitudinal axis, so the longitudinal axis is an axis of symmetry.

Preferably, in order to enable handling and processing of the container in conventional filling facilities and with conventional technology used for filling and processing of standardized syringes, the container may meet, in terms of shape and dimensions, selected specifications of ISO 11040-4 standard of a prefilled syringe. Specifically, all cross-sections perpendicular to its longitudinal axis or axis of symmetry may be circles. Especially in this regard, the container may comprise or form a flange portion or flange along its open second end.

The following three paragraphs give preferred dimensions according to various standards the container may comply with. Following the terminology used in these standards, the container without flange portion is called a barrel.

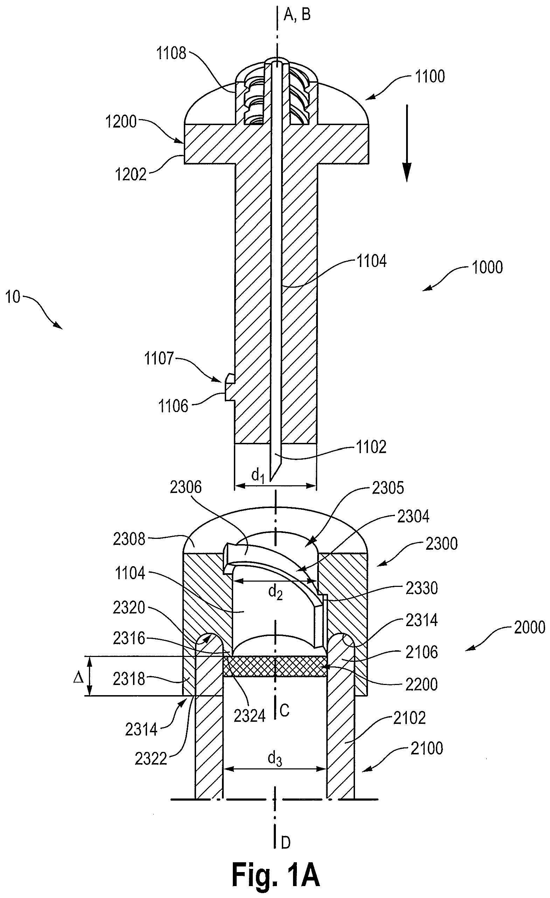

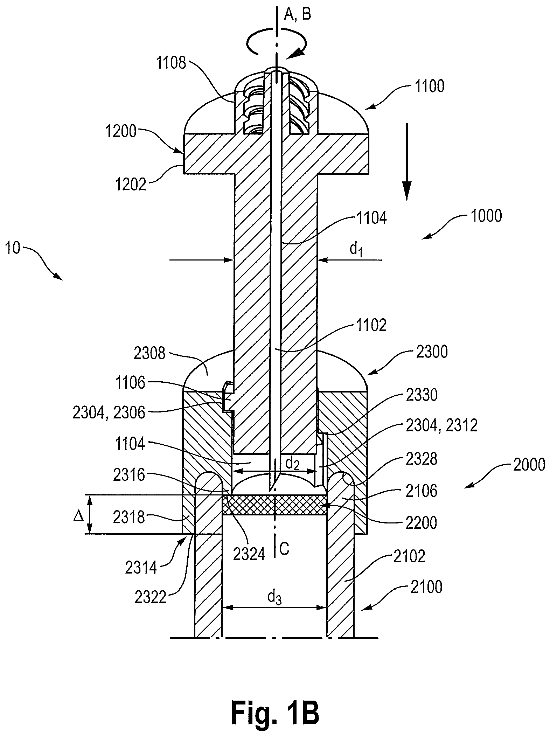

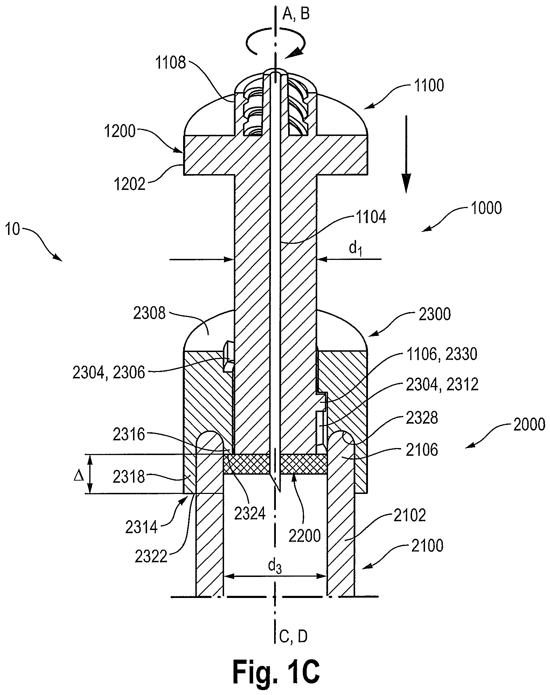

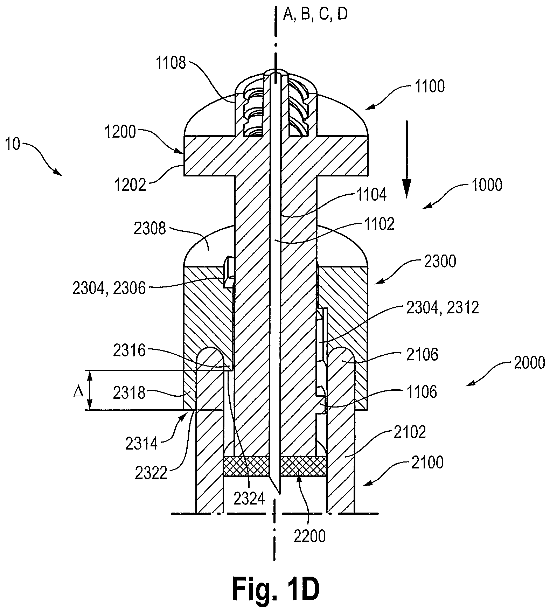

Preferably the cylindrical barrel complies, in terms of its inner diameter, outer diameter and wall thickness, with the relevant specifications of the above-mentioned ISO 11040-4 standard for a suitable specific standardized nominal volume. The specific standardized nominal volume may correspond to or may be close to, i.e. may slightly differ from, the predetermined filling volume of the container, i.e. is a suitable one of the various nominal volumes considered in the ISO 11040-4 standard. In particular, the cylindrical barrel may be formed to meet the barrel diameters d1 and d2 and the barrel wall thickness s1 as indicated in Figure 1 and Table 1 of the ISO 11040-4 standard for a specific standardized nominal volume of a standardized syringe. Depending on the predetermined filling volume, the length of the barrel may conform to the length l1 or total length l of a standardized syringe as indicated in Figure 1 and Table B.1 of ISO 11040-4 standard, or may vary within a range defined by the length l1 and the total length l as aforementioned, or may even be different from the specifications of the ISO 11040-4 standard. Generally, the length of the cylindrical barrel is defined and set so that the predetermined filling can be achieved with the inner diameter, outer diameter, and wall thickness adopted from the ISO 11040-4 standard for the specific nominal volume as above explained. If the predetermined filling volume matches a standardized nominal volume of a standardized syringe, the standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this standardized or specific nominal volume. In this case, the length of the barrel may meet the length 11 indicated as indicated in Figure 1 and Table 1 of the ISO 11040-4 standard for the standardized nominal volume. If the predetermined filling volume differs from any of the nominal volumes set by the ISO 11040-4 standard, any suitable nominal volume close to the predetermined filling volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this suitable or specific nominal volume. Even if the predetermined filling volume matches a standardized nominal volume of a standardized syringe, however, any other suitable standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this other standardized or specific nominal volume. For example, if the predetermined filling volume is 1 ml the cylindrical barrel may meet the outer diameter d1, inner diameter d2, and wall thickness s1 of a 1 ml syringe in a long version, with d1 being 8.15 mm ±0.1 mm, d2 being 6.35 mm ±0.1 mm, and s1 being approximately 0.9 mm (cf. ISO 11040-4, Tables 1 and B.1 for a nominal volume of 1 ml). In this case, the barrel length may meet the length l1 of the 1 ml syringe in the long version, being 54 mm ±0.5 mm, with the specific nominal volume being 1 ml, With the same predetermined filling volume of 1 ml, however, the cylindrical barrel may alternatively meet the outer diameter d1 (= 10.85 mm ±0.1 mm), inner diameter d2 (= 8.65 mm ±0.2 mm), and wall thickness s1 ≈ 1.1 mm) of a 1 ml syringe in a short/standard version (cf. ISO 11040-4, Tables 1 and B.1 for a nominal volume of 1 ml) with the specific nominal volume being 1 ml, or may meet the outer diameter d1 (= 6.85 mm ±0.1 mm), inner diameter d2 (= 4.65 mm ±0.1 mm), and wall thickness s1 (≈ 1.1 mm) of a 0.5 ml syringe (cf. ISO 11040-4, Tables 1 and B.1 for a nominal volume of 0.5 ml) with the specific nominal volume being 0.5 ml. In these alternative cases, the barrel length is appropriately adjusted so as to ensure that the container provides the predetermined filling volume.

Further, the above mentioned flange portion put on top of the cylindrical barrel may form, in a circumferential direction, a continuous circular flange in line with the flange of a conventional vial according to the ISO 8362-1 standard. Insofar, the flange portion may be in line with a form B of a finger flange of a standardized syringe (cf. ISO 11040-4, Figure 1, Form B). Additionally, the flange portion may be formed to comply, in terms of its cross-sectional shape, with relevant specifications of the above mentioned ISO 8362-1 standard. In particular, the flange portion may meet, in terms of its axial length/height, in terms of its upper inner edge, and/or in terms of its upper end surface, the relevant specifications as indicated in Figure 1, Figure 2, Figure 3 and Table 1 of the ISO 8362-1 standard. Specifically, the axial length/height of the flange may amount to 3.6 mm ±0.2 mm, the bevel angle of the upper inner edge may be approximately 45°, and/or the taper angle of the upper end surface of the flange may be 3° ±2° (cf. ISO 8362-1, Figures 1 to 3). While the above mentioned 3.6 mm ±0.2 mm adopted from the ISO 8362-1 standard are preferable, the axial length/height of the flange portion may slightly differ from this standardized dimension as long as the flange portion still meets the following two functions: first, the flange portion shall enable handling and processing the container in conventional filling facilities and with conventional technology used for filling and processing of standardized syringes, i.e. the flange portion shall meet the function of a finger flange of a standardized syringe (cf. ISO 11040-4, Figure 1); and secondly the design of the flange portion shall allow a precise fitting of the container to the extraction element. Further, the flange portion may differ, in terms of its inner diameter, outer diameter and its lower end surface, from the relevant specifications of the ISO 8362-1 standard (cf. ISO 8362-1, Figure 1: diameter d4, diameter d2) in order to enable the flange portion to smoothly match the respective barrel dimensions. In particular, the flange portion may have a radially extending flat lower end surface unlike a standardized vial which has a tapered lower surface (cf. ISO 8362-1, Figures 1 to 3 showing a taper angle of 10° ±5°). A flat lower end surface may facilitate the handling of the container in conventional filling facilities and with conventional technology used for filling and processing of standardized syringes. Generally, however, the lower end surface of the flange portion may be formed with a taper angle as it is known from ISO 8362-1 (cf. Figures 1 to 3 showing a taper angle of 10° ±5°).

Accordingly, unlike a conventional vial the container of the container unit, in particular if used as a primary packaging, according to the present invention may have, at its outer surface, no such neck constriction as it is seen and specified in the ISO 8362-1 standard (cf. ISO 8362-1, Figures 1 to 3: diameter d3, height h3). On the other hand, the inner surface of the container may be finished at the upper end, i.e. the upper end section oppositely to the flange portion, in line with the finish of any appropriate one of vial models A, B, or C of the ISO 8362-1 standard (cf. ISO 8362-1, Figures 1 to 3). Specifically, the inventive flange may at least be as thick as that of a standard prefilled syringe.

The closure element is accommodated within the container in a fluid-tight contact with the circumferential wall. In other words, the closure element is held by friction within the container. To this end, the closure element may preferably be formed so as to be elastically deformable. That is, the closure element, prior to being penetrated by the hollow needle in the process of delivery of the compounds contained in the container, fluid-tightly (i.e. compound-tightly and, notably, antiseptically) seals the container by forming a leak-proof circumferential contact with its circumferential inner surface.

In a preferred embodiment, once the closure element has been penetrated and the extraction unit is in abutting contact with the closure element, the closure element may be pushed by the extraction unit, preferably by the penetration and discharge means, against the frictional force towards the container bottom to displace and discharge the medical or pharmaceutical compound from the container into the hollow needle. Accordingly, in this preferred embodiment the extraction unit, preferably the penetration and discharge means, may serve as a piston adapted to slide or move the closure element towards the container bottom to displace and discharge the medical or pharmaceutical compound from the container into the hollow needle.

In an alternative preferred embodiment, once the closure element has been penetrated and the extraction unit is in abutting contact with the closure element, the closure element may remain with the extraction unit in an initial position, i.e. may not be pushed by the extraction unit against the frictional force towards the container bottom, and the medical or pharmaceutical compound may be drawn from the container into the hollow needle by applying negative pressure to the container via the hollow needle, or may flow out of the container by gravitational force.

It should be noted that according to the present invention in any of the above mentioned embodiments the closure element is penetrated. That is, the fluid-tight contact between an outer circumference of the closure element and the inner surface of the container is such that a force greater than a penetration force for penetrating the closure element is needed to move the closure element towards the container bottom. Therefore, the closure element and the container together form a sort of slide-press-fit. Advantageously, the closure element may have a through hole aligned with the hollow needle and closed on either one side by means of a diaphragm. In such a case, as a matter of course, only the diaphragm has to be penetrated.

The penetration and discharge unit serves to penetrate the closure element thereby to activate the inventive system, i.e. to enable the extraction and delivery of the compounds stored in the container and to finally extract the compounds. To this end, the extraction unit comprises any kind of a penetration and discharge means that has, i.e. supports, holds and/or forms, a hollow needle. By penetration of the closure element, the sterile storage of the compounds is undone at the point of penetration (and only there). In this regard, the hollow needle may at least partially extend through a through hole extending through the penetration and discharge means along the longitudinal axis. Further, as above mentioned the penetration and discharge means may serve to slide or move the closure element towards the container bottom thereby to displace and discharge the medical or pharmaceutical compound from the container into the hollow needle. In this regard, the penetration and discharge has an elongated structure with a cross-section smaller than a cross-section of the closure element and of the container, in order to plunge or enter into the container.

Further, the hollow needle may at least partially extend through a through hole of the hollow needle supporting portion. That is, the hollow needle may preferably extend to only such an extent into the penetration and discharge means that a save support is assured or may extend completely through the through hole, projecting out of it or not at the other end of the hollow needle supporting portion. Advantageously, the through hole in the former case is formed as a stepped hole, having a first portion accommodating the hollow needle, and a second portion having a diameter essentially equal to the inner diameter of the hollow needle, in order for a fluid channel formed within the hollow needle supporting portion to have an essentially constant diameter.

The inventive hollow needle serves the penetration of the closure element, and, to this end, protrudes or projects or juts out from an end face of the penetration and discharge means opposite or facing the container bottom parallel to, preferably aligned with, the longitudinal axis. Specifically, the hollow needle is firmly held by the penetration and discharge means, preferably extending into it by a predetermined amount assuring its firm position specifically in the process of penetration. It should be emphasized that herein the term hollow needle refers to the hollow needle used to penetrate the closure element, whereas the term cannula used later refers to a hollow needle for application or administration of the compounds, that is arranged on a patients side, for example.

The link motion is defined between the first and the second link motion portions of the extraction unit, preferably the penetration and discharge means, and the container, respectively. Specifically, the first link motion portion is provided at the penetration and discharge means, while the second link motion portion is provided at the container. One of the link motion portions may be provided with one of the projection and the guiding groove, while the other of said link motion portions may accordingly be provided with the other of the projection and the guiding groove. Accordingly, said extraction unit is coupled to said container by means of the link motion, i.e. by means of the first link motion portion and second link motion portion. In other words, the link motion is a form-locking connection the extraction unit forms with the container. The form-locking connection realized by the link motion between the first link motion portion and the second link motion portion shall prevent an unintended or accidental separation of the extraction unit, more specifically of the penetration and discharge means having the hollow needle, and the container.

The inventive link motion is a deconnectable or unlockable or removable link or connection between the penetration and discharge means and the closure element, where either one of them is provided with a slide block, i.e. a pin or peg - that is, the inventive projection -, that is moved along a predetermined trajectory by the guiding groove arranged in the respective other one of them. In particular, this implies that the inventive motion is not a screw joint.

It should be noted that the term link motion is sometimes and synonymously called motion link, both terms referring to a structure, not to a type of motion, as the first term might suggest.

Specifically, the motion of one of the first and second link motion portions as a whole with respect to the other one of the first and second link motion portions follows the guiding groove. This may be achieved if the projection as part of the one link motion portion is an element that is unflexible and steadfastly connected, preferably integrally connected, to a main body of the one link motion portion. Alternatively, the projection may in principle be flexibly connected to the main body of the one link motion portion in the way of any conventional ballpoint mechanism where a ballpoint refill is linearly movable while a projection of a compression piece thereof, usually activated by a thumb of a user to move the refill in and out, is flexible. Usually, in such a ballpoint mechanism, the compression piece is pressed once to move out the refill, and is removed in by pressing the compression piece a second time. Therefore, according to a preferred aspect of the present invention, the link motion may be designed in line with such a ballpoint mechanism.

Especially, in the latter case, the extraction unit may preferably be elastically biased relative to the container in a direction opposite to the needle penetration direction. The needle penetration direction is the moving direction of the first link motion portion, i.e. of the penetration and discharge means and of the hollow needle, with respect to the second link motion portion, i.e. the container, and the closure element. An elastic force biasing the extraction unit in a direction opposite to the penetration direction may be achieved by an elastic restoring force of the closure while penetrating the closure element. Additionally or alternatively, such an elastic force may be achieved by providing an elastic element, e.g. a compression spring, between the extraction unit and the container or, in functional terms, between the first link motion portion and second link motion portion.

The shape of the guiding groove and, therefore, the trajectory of the projection or the predetermined way, so to speak, is not specified in any way. That is, the guiding groove may generally have the shape of a regular spiral, i.e. a helical curve, or may be curved in any other way, or may be just straight, or may form spiral, curved and/or straight guiding groove sections. In other words, the guiding groove may extend (linearly) along and/or (rotationally) around the longitudinal axis so as to define a predetermined way of movement of the projection, which extends linearly along and/or rotationally around the longitudinal axis.

A straight guiding groove is the simplest form of a guiding groove and may nevertheless have the advantage that the penetration and discharge means can not be retracted once the closure element has been penetrated. Therefore, the delivery system can be depolluted as a whole, without the danger of contaminating something with its former compounds.

According to an advantageous aspect of the present invention (claim 2), the first link motion portion is arranged at the penetration and discharge means.

According to a further advantageous aspect of the present invention (claim 3), the first link motion portion may be inserted into the second link motion portion.

In other words, the second link motion portion may be formed as a corresponding recess or blind hole provided in the container, receiving the first link motion portion projecting from the extraction unit, preferably from the penetration and discharge means. To be specific, in a broadest sense the present invention merely requires that the link motion is formed between the first and second link motion portions. Since any link motion composed of two components implies that one of these is inserted into the respective other one, this advantageous aspect defines the specific spatial relationship shown in Fig. 1A, for example. Alternatively, the closure element could be formed such that the second link motion portion extends into the penetration and discharge means, i.e. such that the first link motion portion is formed as a hole. In both cases, the projection can be either in the penetration and discharge means or in the attachment element.

According to a further advantageous aspect of the present invention (claim 4), the second link motion portion is arranged in an attachment element attached to a rim portion of the container, which rim portion forms the open second end.

The attachment element serves to connect, mount or fit the extraction unit to the container and may, therefore, be regarded as an adapter that is part of the container unit. Therefore, the attachment element also provides a function of dimensional adaptation allowing the design to be individually optimized to some extend. Furthermore, the attachment element assures an appropriate alignment of the extraction unit with respect to the container to be attached to, and - by its link motion portion - a guidance in the process of attachment of the extraction unit and in the process of penetration of the closure element by the hollow needle.

According to a further advantageous aspect of the present invention (claim 5), the penetration and discharge means extends through a through hole of the attachment element, wherein the penetration and discharge means and the through hole are radially dimensioned to form a loose fit.

That is, the attachment element may be formed as a ring-shaped cap surrounding the penetration and discharge means.

When, alternatively, diameters are used to define the dimensions, the diameter of the penetration and discharge means is smaller but essentially equal to the diameter of the through hole, where the diameter is taken in a plane perpendicular to the longitudinal axis and the guiding groove and the projection are disregarded. Therefore, it is assured that the link portion is stably positioned by and guided within the through hole of the attachment portion.

According to a further advantageous aspect of the present invention (claim 6), the attachment element has a first overlapping portion outwardly overlapping the container, while according to a further advantageous aspect of the present invention (claim 7), the attachment element has a second overlapping portion inwardly overlapping the container.

That is, in a longitudinal section including the longitudinal axis of the container, the attachment element overlaps the container in a U-shape in case of inwardly and outwardly overlapping the container, where the legs of the U (the I-portions thereof) may be of equal of different length.

According to a further advantageous aspect of the present invention (claim 8), at least the first overlapping portion may be of an elastic material.

That is, due to its elasticity, the first overlapping portion can be put or pulled over the open second end of the container in a tautened or stretched state thereof to assure a tight but detachable press-fit. Preferably, the first overlapping portion is made of rubber or the like.

According to a further advantageous aspect of the present invention (claim 9), a portion of the attachment element forming a part of the second link motion portion is made of a non-elastic material.

The non-elastic material of that part assures its form stability and, therefore, an easy rotational-translational movement of the penetration and discharge means (specifically the hollow needle supporting portion) with respect to and within the attachment element. Preferably, the contact surfaces of both components are smooth and their respective materials selected in such a way that the friction between is minimum. Alternatively, the attachment element may comprise an appropriate inset that advantageously is glued into a main body of the attachment element and is made of the non-elastic material to provide the mentioned advantage.

According to a further advantageous aspect of the present invention (claim 10), the extraction unit comprises a grip portion connected to the penetration and discharge means.

Preferably, the penetration and discharge means and the grip portion are integrally formed or formed in a one-piece fashion. Preferably, the grip portion is provided with a knurled-like surface having indentations and/or projections that allow a firm holding of the penetration and discharge means in the process of penetration of the closure element by the hollow needle. Preferably, an outer circumference of the grip portion has a shape of a circular cylinder. Alternatively, the shape is polygonal in cross-section. The hollow needle supporting portion serves to firmly hold the hollow needle in a well-defined position, as is already noted above, wherein the firm hold is achieved by press-fitting or glueing the hollow needle into a through hole extending through the penetration and discharge means.

According to a further advantageous aspect of the present invention (claim 11), the extraction unit has a delivery end formed as a part of a Luer lock connector.

The Luer lock connector is just one preferred (because of its standardization) inventive design of a connector connecting the inventive delivery system to another (or other) device(s) or a human body or a body of an animal. The part of a Luer lock connector that is part of the inventive delivery system may be formed as either the female or the male part thereof.

According to a further advantageous aspect of the present invention (claim 12), the extraction unit alternatively has a delivery end formed as a spray valve.

According to this advantageous aspect, there is no cannula. Instead, a blunt end portion of the hollow needle (the end portion opposite to the lower (in the figures) sharp end portion) advantageously transitions or blends into the spray valve.That is, the blunt end portion of the hollow needle is formed as the spray valve. Alternatively, the spray valve is a separate component, attached to or inserted into the channel extending through the penetration and discharge means. Advantageously, the spray valve is recessed or set back into an upper or outer surface of the penetration and discharge means in order not to protrude therefrom.

According to a further advantageous aspect of the present invention (claim 13), extraction unit alternatively has a delivery end formed as a cannula.

A cannula is a hollow needle adapted to be plugged on a syringe to put a medical fluid into a human being or an animal, mostly intravenously. Preferably, the cannula is of a type that allows the use of the inventive system as an infusion system, for example, i.e. allows a flow of air into the container and, thereby, a flow of the compounds out of the container solely by gravitation. The term to extract used above may in this case be reasonably replaced by to empty in view of the passive draining of the compounds, i.e. the draining without the help of some kind of actuator.

According to a further advantageous aspect of the present invention (claim 14) and as above suggested, the container has a flange extending along the open second end, and the above mentioned first overlapping portion of the attachment element has a circular groove adapted to accommodate the flange.

That is, the ring-shaped flange extends outwardly into the groove being essentially, that is e. g. in all matters relating to a reliable fit, complementarily shaped. In case the first overlapping portion is rubber-like, it can be put over the flange while the latter snaps into the groove. The attachment according to this advantageous aspect improves the fit of the cap on the container, i.e. the connection between both.

According to a further advantageous aspect (claim 15) concerning the above mentioned preferred embodiment of the present invention where the closure element is pushed by the penetration and discharge means towards the container bottom, contact surfaces of the container bottom and the closure element may be complementarily shaped, and at least one of the contact surfaces comprises a blind hole for accommodating a tip of the hollow needle.

The tip of the hollow needle is accommodated in the blind hole in a situation allowing a force along the longitudinal axis to be transmitted from the penetration and discharge means to the closure element to move the closure element towards the container bottom. Complementarily shaped means herein that the opposing surfaces of the closure element and the container bottom are configured such that they are essentially in surface contact with each other when the piston portion (penetration and discharge means) is located at its most downward position (i.e. when the compounds initially stored in the container have been extracted as much as possible with the hollow needle in its complete extraction allowing position), where essentially means except for a depression or recession (blind hole) that is formed in one or both of them. This implies that the contact surfaces are essentially parallel to each other (except for the depression). That is, any convexity of one of the contact surfaces corresponds to the concave mirror image of the other one of the contact surfaces.

According to a further advantageous aspect of the present invention (claim 16), the contact surfaces are generally flat-shaped or ellipsoidically or spherically shaped.

The shapes of the contact surfaces are advantageous variations in terms of their respective manufacturing process as well as for the extraction efficiency they allow. However, in principle, the contact surfaces may be shaped arbitrarily. Preferably, the ellipsoidically or spherically shaped contract surfaces are disposed such that their respective axis of symmetry is aligned with the longitudinal axis.

According to a further advantageous aspect of the present invention (claim 17), in any of the above mentioned preferred and alternative preferred embodiments of the present invention, the guiding groove (i.e. the predetermined way, may extend along (i.e. linearly along) and/or around (i.e. rotationally around) the longitudinal axis.

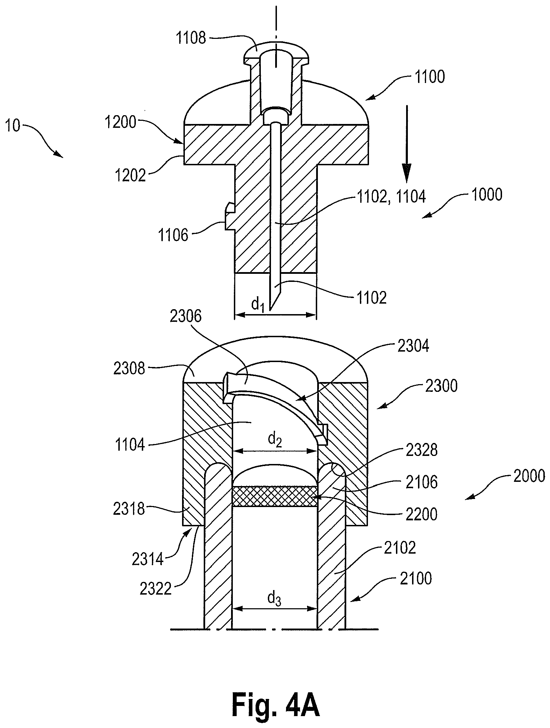

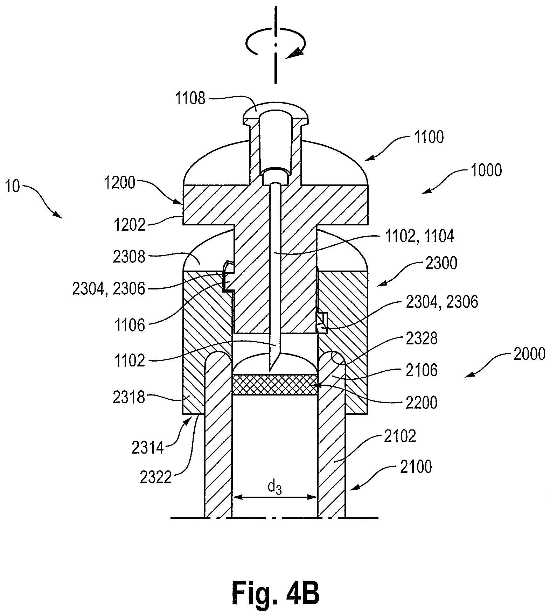

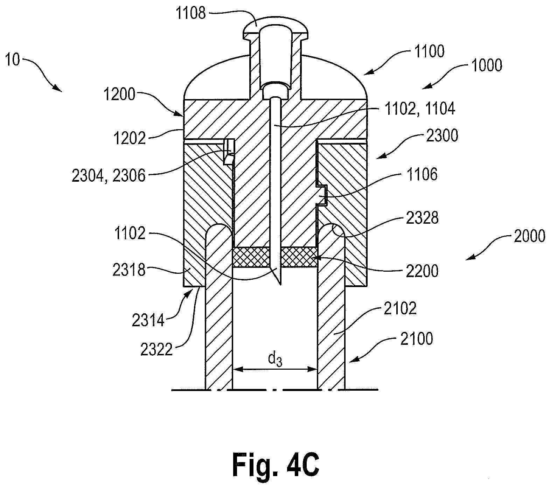

In this regard (claim 18), the guiding groove may be such that a relative movement of the penetration and discharge means with respect to the mounting element is carried out by (i) rotating and/or (linearly) advancing the extraction unit along the longitudinal axis to longitudinally move the hollow needle by a first distance h2 to make the hollow needle penetrate the closure element, and (ii) further rotating the extraction unit to longitudinally move the hollow needle by a second distance (h2 - h1) to retract the hollow needle to a complete extraction allowing position. Here, the complete extraction allowing position is a position that allows to extract essentially all of the compounds stored in the container to be extracted. First of all, this position is determined in that, in the penetration process, the hollow needle has to completely penetrate the closure element. Depending on the structure of the closure element, the closure element has to be over-penetrated to some extent. In the case of a diaphragm, as mentioned above, the diaphragm may be stretched and spring back. This over-penetration position is the position achieved by the first distance (h2). According to the present advantageous aspect, the hollow needle is then retracted to the complete extraction allowing position (as for terminology, the over-penetration position may be called a complete penetration position). This position is achieved by the second distance (h2- h1, with h1 < h2). A retraction of the hollow needle may serve to achieve a more or less complete extraction of the medical or pharmaceutical compound from the container. Where the closure element is not moved towards the container bottom, e.g. in case of the above mentioned alternative preferred embodiment, cf. e.g. the embodiment shown in Figs. 4A to 4D), a retraction of the hollow needle may assure that the hollow needle does not extend beyond the closure element towards the container bottom. Preventing such extension of the hollow needle beyond the closure element may facilitate a more or less complete extraction of the medical or pharmaceutical compound from the container.

In this regard (claim 19), the guiding groove may further be formed such that a relative movement of the extraction unit with respect to the container unit is further carried out by (iii) rotating the extraction unit to a locking position, preferably without any further longitudinal movement of the hollow needle. The longitudinal locking position is a position in which the extraction system is longitudinally fixed. In the variation where the closure element is to be moved, this position may also be called a longitudinal force transmittable position allowing a longitudinal force, i.e. a force along the longitudinal axis sufficient to move the closure element to be applied. Thus, in a longitudinally locked state, the penetration and discharge means, i.e. the extraction unit, is secured to the container unit.

According to a further advantageous aspect of the present invention (claim 20), the delivery system is a modular system comprising the extraction unit as a first module and the container unit as a second module.

Advantageously, therefore, the first and second modules can be exchanged in case one of them is damaged or in case differently shaped containers and/or containers storing different compounds and/or differently shaped extraction units are to be used or in case one of them is to be cleaned in the sense of sterilized, as long as they are correspondingly shaped to be connectable which is assured by the attachment element. Preferably, therefore, a user may order only a single first module used for a plurality of different compounds.

According to a further advantageous aspect of the present invention (claim 21), the second module may comprise the container, the attachment element, and the closure element as a first, a second, and a third sub-module, respectively.

According to a further advantageous aspect of the present invention (claim 22), at least one of the modules may be sterilizable.

According to a further advantageous aspect of the present invention (claim 23), the extraction unit is elastically biased in a direction away from the container unit.

According to a further advantageous aspect of the present invention (claim 24), the delivery system comprises an elastic element arranged between the extraction unit and the container unit.

Further features and advantages of the present invention will become apparent from the following detailed description of preferred embodiments with reference to the accompanying drawings. In the drawings:
Figs. 1A to 1D are schematic drawings showing a delivery system according to a first embodiment of the present invention;
Figs. 2A to 2D are schematic drawings showing a delivery system according to a second embodiment of the present invention;
Figs. 3A to 3C are schematic drawings showing variations of a closure element and a container bottom of a delivery system according to the first and second embodiments;
Figs. 4A to 4D are schematic drawings showing a delivery system according to a third embodiment of the present invention;
Fig. 5A and 5B are schematic drawings of a developed surface of a link motion according to a delivery system according to the present invention;
Fig. 6 is a schematic enlarged view of an alternative container having a flange portion;
Fig. 7A to 7C are schematic drawings showing variations of a delivery system according to the present invention;
Figs. 8A and 8B are schematic drawings illustrating an alternative of a link motion of a delivery system according to the present invention;
Figs. 8C and 8D are schematic drawings illustrating a modification of the alternative of Figs. 8A and 8B; and
Figs. 1A to 1D schematically show a delivery system 10 according to a first embodiment of the present invention.

The delivery system 10 comprises an extraction unit 1000 and a container unit 2000.

The extraction unit 1000 comprises a penetration and discharge means 1100, and a grip portion 1200. The container unit 2000 comprises a container 2100, a closure element 2200, and an attachment element 2300.

The penetration and discharge means 1100 serves as a hollow needle supporting portion and comprises a hollow needle 1102 mounted in a through channel or through hole 1104 thereof in such a way that a longitudinal axis A of the penetration and discharge means 1100 and a longitudinal axis B of the hollow needle 1102 coincide. The hollow needle 1102 has a lower, chamfered end portion and an upper end on a level with an upper end face of the penetration and discharge means 1100.

The penetration and discharge means 1100 is further provided with a projection 1106 as part of a first link motion portion 1107.

An upper end portion 1108 of the penetration and discharge means 1100 is formed as a male part of a Luer lock connection, having a central, arbor-like projection housing an upper portion of the channel 1104 accommodating the hollow needle 1102.

The grip portion 1200 integrally, i.e. in one-piece fashion, formed to the penetration and discharge means 1100. As shown in Fig. 1A, the grip portion 1200 is a ring surrounding the penetration and discharge means 1100 or, to put it differently, extending laterally (i.e. perpendicularly to the longitudinal axis A) outwardly from the penetration and discharge means 1100. The grip portion 1200 has a gripping surface 1202 as a circular, band-shaped outer lateral (in the figures) surface, advantageously textured in some way (not shown) to improve the grip or hold by hand, when the extraction unit 1000 is turned into the attachment element 2300 described below. It should be noted that elliptical lines are drawn in the figures to visualize schematically a slight tilt of the upper surfaces to demonstrate their respective forms. The sections shown are not affected therefrom.
The container 2100 is of a hollow right-cylindrical shape and comprises a tube-shaped side wall 2102 and a container bottom 2104 closing the side wall 2102 at a lower end in Fig. 1. The side wall 2102 has a ring-shaped upper end portion 2106 forming a open second end of the container 2100.
The attachment element 2300, being part of the container unit 2000, is a generally ring-shaped, cap-like adapter provided with a through hole 2302 and a guiding groove 2304 as part of a second link motion portion 2305, which guiding groove 2304 extends along a surface of the through hole 2302 (an inner surface of the attachment element 2300). The projection 1106 is adapted to be engaged with and glide within the guiding groove 2304 to form a link motion to allow for a well defined rotational-translational movement of the penetration and discharge means 1100, i.e. of the extraction unit 1000 as a whole, with respect to the attachment element 2300, i.e. the container 2100, in a predetermined way towards the container bottom 2104. The guiding groove 2304 in this embodiment comprises a spiral groove portion 2306 that extends from a circular, ring-shaped upper surface 2308 of the attachment element 2300 to an inner point 2310 thereof, spirally winding around the axis A, and a straight groove portion 2312 continuously connected to the spiral groove portion 2306 at the inner point 2310 and extending parallel to an axis C of the attachment element 2300 to a lower surface 2314 thereof, wherein the axis C coincides with the axes A, B in a connected state of the penetration and discharge means 1100 to the attachment element 2300, as shown in Figs. 1B and 1C.
As shown in Fig. 1A, a lower end portion of the attachment element 2300 is shaped such that it straddles the end portion 2106 of the container 2100, wherein a ring-shaped inner portion 2316 longitudinally (along the axis C) extends less along an inner surface of the container 2100 towards the container bottom 2104 than a ring-shaped outer portion 2318 extends along an outer surface of the container 2100. Therefore, there is a longitudinal difference Δ between a circular lower edge of the inner portion 2316 and a circular lower edge of the outer portion 2318. As shown in Fig. 1A, the inner portion 2316 and the outer portion 2318 form a circular groove 2320 with a half-pipe-like roof (or bottom, when regarded upside-down compared to Fig. 1A, so that the circular groove 2320 forms a circular trough with walls of different wall heights). Therefore, the lower surface 2314 of the attachment element 2300 is a sum of an outer ring-shaped end face 2322, an inner ring-shaped end face 2324, and a surface 2328 of the roof. The circular groove 2320 serves to accommodate the end portion 2106 of the container 2100.

The closure element 2200 is formed from an elastic material and accommodated within the container 2100 in a fluid-tight contact with the container to be adapted to seal the container 2100 until delivery of the compounds stored therein. In a position as shown in Fig. 1A, that is in its not-yet down-shifted or initial position, the closure element 2200, which is formed in this embodiment as a circular, slightly elastic disc, is in contact with the end face 2324, and - due to its elasticity - fluid-tightly abuts or rests against the inner surface of the container 2100. In the situation shown in Fig. 1A, the penetration and discharge means 1100 is not yet coupled to or connected with the attachment element 2300, and the closure element 2200 is not yet penetrated or pierced by the hollow needle 1102, to sterilely hold medical or pharmaceutical compounds (not shown) stored therein. In that situation, the container 2100 together with the closure element 2200 forms a primary packaging as defined above.

It should be noted that an outer diameter d1 of the penetration and discharge means 1100, and an inner diameter d2 of the attachment element 2300 are essentially equal, in order for the penetration and discharge means 1100 to snugly fit into the through hole 2302 of the attachment element 2300. In contrast, due to the inner portion 2316, an inner diameter d3 of the container 2100 is larger than each of the outer diameter d1 of the penetration and discharge means 1100 and the inner diameter of the attachment element 2300.

Figs. 1B to 1C show various stages of the penetration process, that comprises the steps of (1) connecting the penetration and discharge means 1100 with the attachment element 2300 by inserting - while holding the penetration and discharge means 1100 at the grip portion 1200 - the projection 1106 into the upper end of the spiral groove portion 2306 of the guiding groove 2304 (Fig. 1B), (2) turning the extraction unit 1000 to thereby move the penetration and discharge means 1100, due to the link motion now established between the penetration and discharge means 1100 and the attachment element 2300, rotatingly along the longitudinal axes A to D (now coinciding), and completely penetrate the closure element 2200 by means of the hollow needle 1102 until the lower surface of the penetration and discharge means 1100 comes in contact with an upper surface of the closure element 2200, and the hollow needle 1102, having penetrated the closure element 2200, projects from the lower surface of the closure element 2200, wherein in that contact position, the projection 1106 is located at point 2330, i.e. the point of continuous connection of the spiral groove portion 2306 and the straight groove portion 2312 (Fig. 1C).

In the stage shown in Fig. 1C, the penetration process is completed, i.e. the elliptical chamfered end face of the hollow needle 1102 is visible below the closure element 2200. By further longitudinally moving the penetration and discharge means 1100 in a downward direction in the figures, while in the beginning of that movement, the projection 1106 is guided by and within the straight groove portion 2312, the penetration and discharge means 1100, acting as a piston portion, shifts the closure element 2200 towards a container bottom 2104 of the container 2100, thereby extracting the compounds stored therein in a flowing direction opposite to the movement direction and delivering them out of the delivery system via the hollow needle 1102.

According to the first embodiment, the upper end portion 1108 formed as a male part of a Luer lock may be coupled to a female part (not shown) of an appropriate device to finally administer the compounds to a patient, for example.

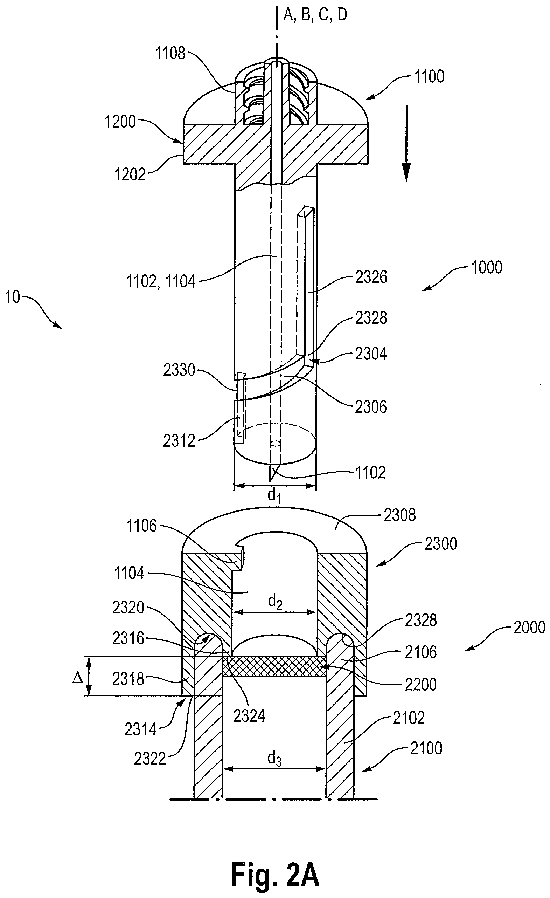

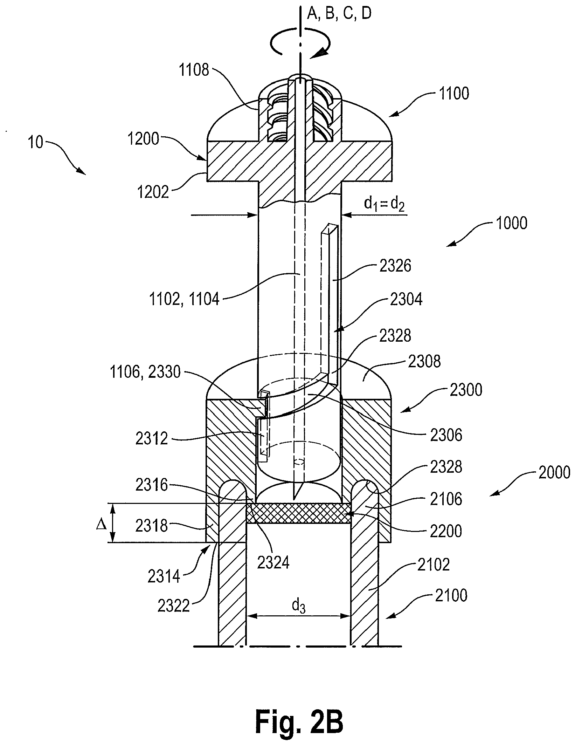

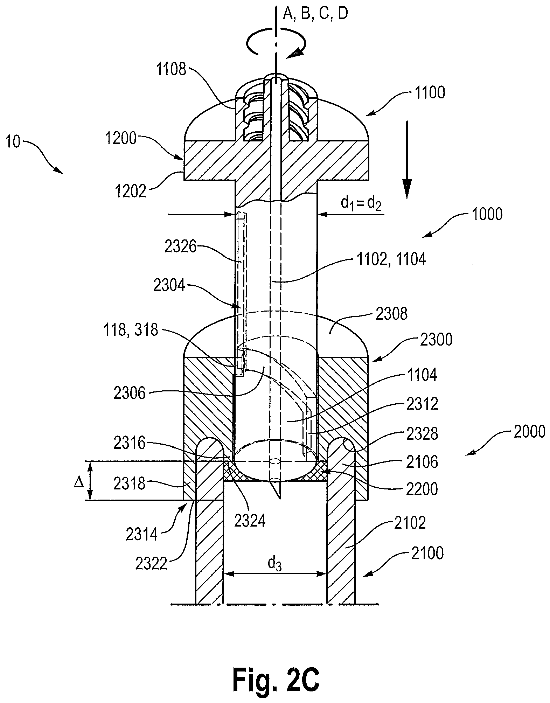

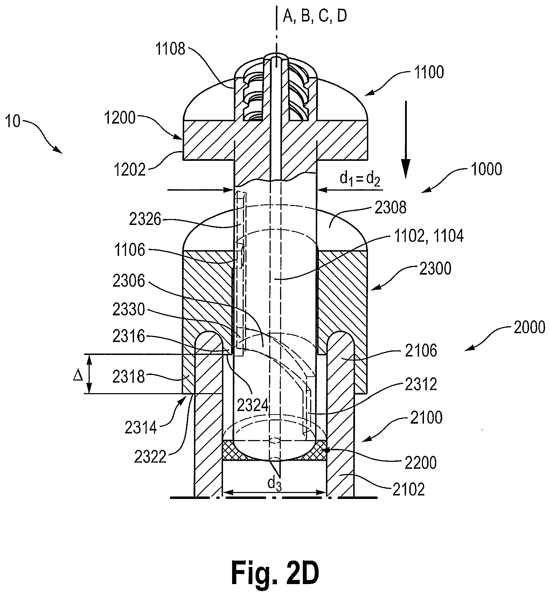

Figs. 2A to 2D show stages corresponding to the stages shown in Figs. 1A to 1D, respectively, of a delivery system according to a second embodiment of the present invention. The second embodiment differs from the first embodiment in that the guiding groove 2304 and the projection 1106 are exchanged. That is, the guiding groove 2304 is formed in the penetration and discharge means 1100, whereas the projection 1106 is formed at the surface of the through hole 2302. In order to emphasize that their respective tasks are identical, their reference numerals are maintained.

In order for the penetration and discharge means 1100 to be able to be connected to the attachment element 2300, and for the penetration and discharge means 1100 to be able to be moved downwardly within the container 2100, the guiding groove 2304 is slightly modified. That is, the guiding groove 2304 is prolonged with respect to the version of the first embodiment by an upper straight groove portion 2326 that is connected to the spiral groove portion 2306 at an upper connecting point 2328 therebetween, and a lower straight groove portion 2312 that is connected to the spiral groove portion 2306 at a lower connecting point 2330.

The above described first and second embodiments correspond to preferred embodiments of the present invention where the closure element is pushed by the extraction unit towards the container bottom in order to displace the medical or pharmaceutical compound from the container into the hollow needle.

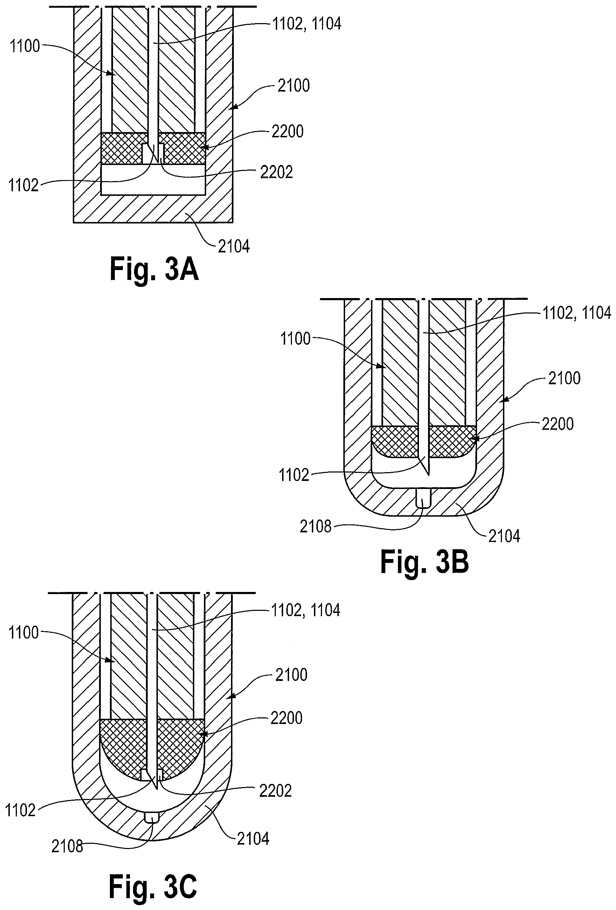

Figs. 3A to 3C show schematic drawings of variations of the container bottom 2104 of the container 2100 and the in terms of shape corresponding closure element 2200 in the first and second embodiments.

As shown in Figs. 3A to 3C, the shapes of contact surfaces of the closure element 2200 and the container bottom 2104 of the container 2100 correspond to each other in that any convex shape of the former is equivalent to a concave shape of the latter. That is, the contact surfaces shown are both flat (Fig. 3A), convex (closure element 2200) and concave with flat surfaces but rounded edges (Fig. 3B), and spherical (Fig. 3C). Furthermore, there is provided a blind hole 2202 in the closure element 2200 and / or a blind hole 2108 in the container bottom 2104 of the container 2100, providing an accommodation space for the tip of the hollow needle 1102 in a state of contact between the respective contact surfaces, in order to reduce the amount of compounds that can not be extracted from the container to a minimum in a completely downshifted state of the penetration and discharge means 1100, and to avoid any damage of the tip (the penetration and discharge means 1100 can be used several times) in the completely downshifted state. It should be noted that in Fig. 3A, the hollow needle 1102 projects from the lower surface of the closure element 2200 at most by an amount equal to a thickness of the closure element 2200, while according to Fig. 3B, the hollow needle 1102 projects from the lower surface of the closure element 2200 at most by an amount equal to a depth of the blind hole 2108 formed in the bottom 204 of the container 2100. The variation shown in Fig. 3C is situated between the above two extremes.

Figs. 4A to 4C show schematic drawings of a delivery system 10 according to a third embodiment of the present invention.

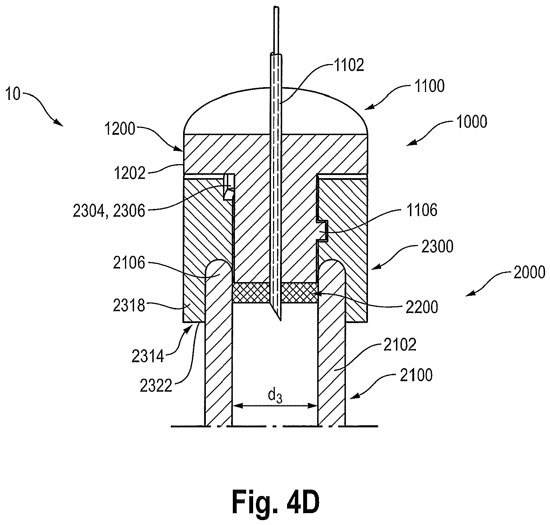

The essential difference between the delivery system 10 of the third embodiment and the delivery systems of the first and second embodiments is that the guiding groove 2304 - here again arranged in the attachment element 2300 - comprises only the spiral groove portion 2306, and does comprise neither the straight groove portion 2312, nor the straight groove portion 2326. Therefore, in the delivery system 10 according to the third embodiment, the penetration and discharge means 1100 does not act as a piston portion: The closure element 2200 is not intended to be shifted towards the container bottom 2104. In contrast, the compounds are extracted from the container 2100 by gravitation, wherein the delivery system 10 of the third embodiment has to be arranged upside-down compared with the figures. To enable air to replace the compounds in the delivery process, either the upper end is formed as part of a Luer lock (her the male part), or the hollow needle 1102 is formed as a double cannula as shown in Fig. 4D.

Therefore, the third embodiment corresponds to an alternative preferred embodiment of the present invention where the closure element remains with the extraction unit in its initial position, i.e. is not be pushed by the extraction unit against the frictional force towards the container bottom, and where the medical or pharmaceutical compound is drawn from the container into the hollow needle by applying negative pressure to the container via the hollow needle, or may flow out of the container by gravitational force.

As shown in Figs. 4A to 4C, diameters d1 to d3, i.e. the outer diameter d1 of the penetration and discharge means 1100, the inner diameter d2 of the through hole 2302, and the inner diameter d3 of the container 2100 may be all equal. Specifically, d2 = d3 implies that the attachment element 2300 according to the third embodiment does not have an inner portion 2316, and the lower surface 2314 is a sum of the lower end face 2322 and the surface 322. The stages shown in Figs. 4A to 4C correspond to the stages of Figs. 1A to 1C and Figs. 2A to 3C.

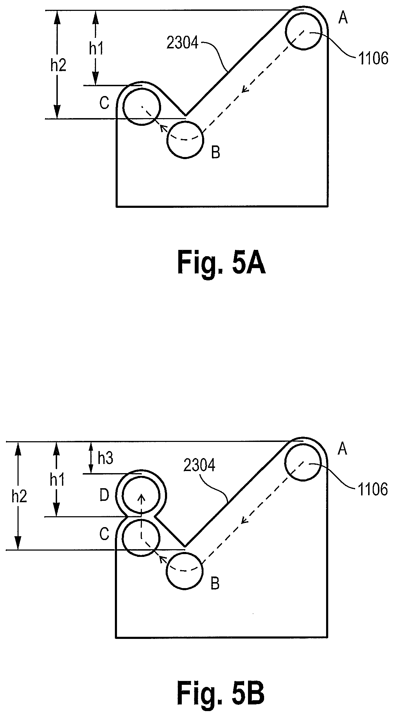

Fig. 5A shows a developed view of a preferred first variation of the link motion, formed by the projection 1106 and the guiding groove 2304. In the developed view, as noted above, the projection 1106 and the guiding groove 2304 may be part of the hollow needle supporting portion 1100 and the attachment element 2300, respectively, or vice versa. That is, in Fig. 5A, the projection 1106 shown may be part of the penetration and discharge means 1100 or part of the attachment element 2300. The same holds correspondingly for the guiding groove 2304. According to Fig. 5A, after the projection 1106 is inserted into or coupled to the guiding groove 2304 at point A, hereafter called the starting position, the projection 1106 is moved along the guiding groove 2304 by relatively rotating the hollow needle supporting portion110 with respect to the attachment element 2300 first downwards by a longitudinal distance h2 to a position B and then upwards by a longitudinal distance h2-h1 to a position C. That is, both distances h1, h2 are measured with respect to the starting position, as shown in Fig. 5A.
Fig. 5B shows a developed view of a preferred second variation of the motion link, formed by the projection 1106 and the guiding groove 2304. The second variation differs from the first variation in that there is added a position D displaced with respect to position C radially and/or longitudinally with respect to the longitudinal axis A. Position D (> h3) is achieved by further relatively rotating the penetration and discharge means 1100 with respect to the attachment element 2300, and is a locking position allowing a longitudinal force to be exerted on the extraction unit 1000 without return-moving the projection 1106 back to position B in the process of pushing the closure element 1200 downwards. As shown in Fig. 5B, h3<h1<h2.

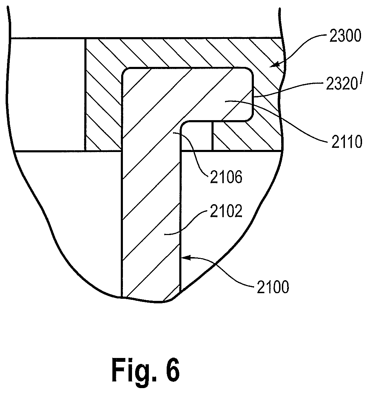

Fig. 6 shows a schematic enlarged detailed view of an alternative container 2100 having a flange 2110 extending outwardly along its rim portion 2106. The flange 2110 is accommodated within a circular groove 2320' of an attachment element 2300', or - to put it differently - in the process of attaching the attachment element 2300' to the container 2100, the attachment element 2300' is stretched and snapped over and around the flange 2110. It should be noted that the attachment element 2300 is drawn very schematically and simplified to emphasize the circular groove 2320, but actually is identically in all other terms with the attachment element 2300 described above.

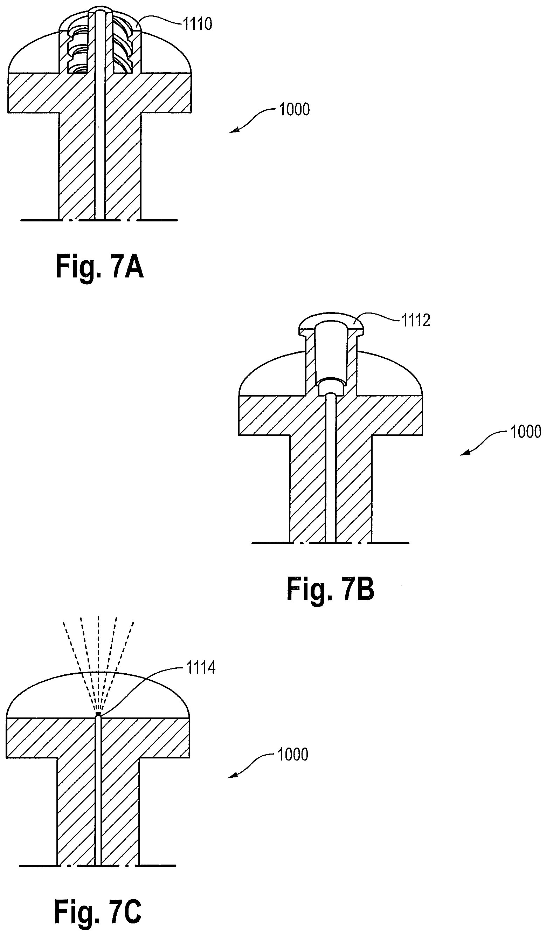

Fig. 7A to 7C each show a variation of the kind of delivery of the delivery system.

The term "kind of delivery" refers to the usage or connectivity of the inventive delivery system 10. Specifically, the delivery system 10 may be coupled by means of a Luer lock connection 1110, 1112 to connect it with an appropriate device, where the Luer lock portion formed at the extraction unit 1000 may be a female portion 1110 as shown in Fig. 7A or a female portion 1112 as shown in Fig. 7B. The hollow needle 1102 may or may not extend up to an upper end face of the Luer lock portion 1110, 1112.

As shown in Fig. 7C, the inventive delivery system 10 may alternatively be used as a spraying device having a spray valve 1114 instead of a Luer lock connection. Pursuant to the modular concept of the present invention, therefore, depending on their respective usage differently shaped extraction units 1000 may be connected to the container unit 2000.

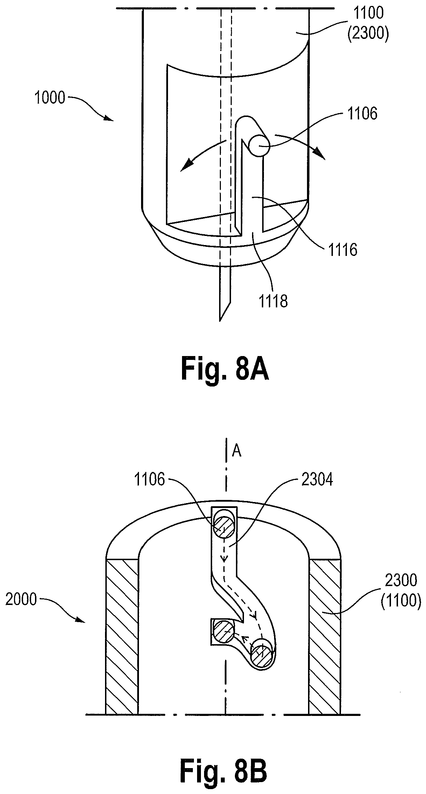

Figs. 8A and 8B are schematic drawings illustrating an alternative or variation of a link motion. In this variation, the link motion follows in terms of structure and functioning a link motion mechanism of a conventional ballpoint as an example. As shown in Figs. 8A and 8B, the projection 1106 may be flexibly connected to a main body of one of a first and a second link motion portion - here either the penetration and discharge means 1100 or the attachment element 2300 - by means of a flexible bar 1116, while the guiding groove 2304 is formed in the respective other one of the first and the second link motion portion - here the penetration and discharge means 1100 and the attachment element 2300. As shown by arrows in Fig. 8A, the flexible bar 1116 is allowed to pivot circumferentially or tangentially with respect to a circumference of the main body in a plane perpendicular to the longitudinal axis about a connection point 1118. The flexible bar 1116 is integrally connected at the connection point 1118 to the main body. The guiding groove 2304 may have the shape as shown in Fig. 8A, resulting in a link motion as known in principle from a ballpoint where its refill (corresponding to the penetration and discharge means 1100) is only linearly movable (here along the longitudinal axis A) while a projection of a compression piece thereof, usually activated by a thumb of a user to move the refill in and out, is flexibly suspended. In the present invention, as in such type of a ballpoint, the compression piece (extraction unit 1000) is pressed once (here moved downwards) to shift out the refill, and is shifted in by pressing the compression piece a second time. Therefore, according to this alternative embodiment of present invention, the link motion is designed in line with such a ballpoint mechanism.

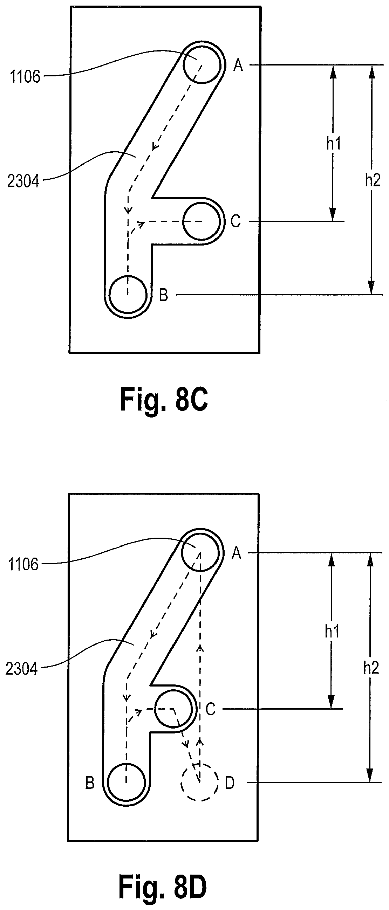

Figs. 8C and 8D are schematic drawings illustrating a modification of the alternative of a link motion of Figs. 8A and 8B. In this modification also, the link motion follows in terms of structure and functioning a link motion mechanism of a conventional ballpoint as an example. The projection 1106 is flexibly connected by means of the flexible bar 1116 (not shown in Figs. 8C and 8D) to the first or second link motion portion, while the guiding groove 2304 is formed in the other one of the first and second link motion portion. When the extraction unit 1000 is pressed a first time, the connection point 1118 of the flexible bar 1116 is moved downwards along a line slightly shifted to the right with respect to a line defined by points A and C. During that movement, the flexible bar 1116 is bent because the projection 1106 is guided within the guiding groove 2304 along a trajectory shown as dashed line from A to B to C. It should be noted that, due to the detailed structure of the mechanism (not shown here) and provided the downward movement is not too slow, the projection 1106, biased by the elastic force of the flexible bar 1116, snaps into the passage to point C only after having reached point B, i. e. in a retracting movement. By pressing the extraction unit 1000 a second time, the projection 1106 (the flexible bar 1116) snaps out of the guiding groove 2304 to allow the extraction unit 1000 to move back along said shifted line. As shown in Fig. 8D, the passage to point C may be shortened compared to that of Fig. 8C. In Fig. 8D, both the movement when the extraction unit 1000 is pressed a first time, and the movement when the extraction unit 1000 is pressed a second time, are each shown by a dashed line having in the direction of the arrows.

It should be noted that all link motions described herein exemplified in Figs. 3A, 3B and 8A to 8D are applicable to the structures of all delivery systems described with reference to the remaining figures.

### Reference Numerals

- 10: delivery system
- 1000: extraction unit
- 1100: penetration and discharge means
- 1102: hollow needle
- 1104: through hole
- 1106: projection
- 1107: first link motion portion
- 1108: upper end portion
- 1110: female part of a Luer lock
- 1112: male part of a Luer lock
- 1114: spray valve
- 1116: flexible bar
- 1118: connection point
- 1200: grip portion
- 1202: gripping surface
- 2000: container unit
- 2100: container
- 2102: side wall
- 2104: container bottom
- 2106: upper end portion
- 2108: blind hole
- 2200: closure element
- 2202: blind hole
- 2300: attachment element
- 2302: through hole
- 2304: guiding groove
- 2305: second link motion portion
- 2306: spiral groove portion
- 2308: upper surface
- 2310: inner point
- 2312: straight groove portion
- 2314: lower surface
- 2316: inner portion
- 2318: outer portion
- 2320: circular groove
- 2322: outer end face
- 2324: inner end face
- 2326: upper groove portion
- 2328: upper connecting point
- A, B, C: longitudinal axes
- d1-d3: diameters

## Claims

1. Delivery system for delivering medical or pharmaceutical compounds, comprising:
- a container unit (2000), comprising:
- a container (2100) for storing said compounds, wherein said container has a circumferential wall (2102), a first end closed by a container bottom (2104) and an open second end (2106), and wherein said container unit (2000) has a longitudinal axis, and
- a closure element (2200) accommodated within said container (2100) in a fluid-tight contact with said circumferential wall, and
- an extraction unit (1000) comprising a penetration and discharge means having a hollow needle (1102) adapted to penetrate said closure element (2200),
wherein:
- said extraction unit (1000) is fitted to said container (2100) so as to be movable relatively to said container (2100) along the longitudinal axis towards the container bottom (2104),
- the delivery system being **characterised in that**:
said extraction unit comprises a first link motion portion (1107), and said container (2100) comprises a second link motion portion (2305), and
- one of said first link motion portion and said second link motion portion is provided with a projection (1106) and the respective other one is provided with a guiding groove (2304), said projection and said guiding groove being engagable to form a link motion adapted to make said extraction unit (1000) move relatively to the container (2100) in a predetermined way towards said container bottom (2104) thereby to make said hollow needle (1102) penetrate said closure element (2200).

2. Delivery system according to claim 1, wherein said first link motion portion is arranged at said penetration and discharge means.

3. Delivery system according to claim 1 or 2, wherein said first link motion portion is inserted into said second link motion portion.

4. Delivery system according to one of the preceding claims, wherein said second link motion portion is arranged in an attachment element attached to a rim portion of said container, which rim portion forms the open second end.

5. Delivery system according to claim 4, wherein said penetration and discharge means extends through a through hole of said attachment element, wherein said penetration and discharge means and said through hole are radially dimensioned to form a loose fit.

6. Delivery system according to claim 5, wherein said attachment element has a first overlapping portion outwardly overlapping said container.

7. Delivery system according to claim 5 or claim 6, wherein said attachment element has a second overlapping portion inwardly overlapping said container.

8. Delivery system according to claim 6 or 7, wherein at least said first overlapping portion is of an elastic material.

9. Delivery system according to any one of claims 4 to 8, wherein a portion of said attachment element forming said second link motion portion is made of a non-elastic material.

10. Delivery system according to one of the preceding claims, wherein said extraction unit comprises a grip portion connected to said penetration and discharge means.

11. Delivery system according to one of the preceding claims, wherein said extraction unit has a delivery end formed as a part of a Luer lock connector.

12. Delivery system according to one of claims 1 to 10, wherein said extraction unit has a delivery end formed as a part of a spray valve.

13. Delivery system according to one of claims 1 to 10, wherein said extraction unit has a delivery end formed as a cannula.

14. Delivery system according to one of claims 6 to 13, wherein said container has a flange extending along said open second end, and said first overlapping portion has a circular groove adapted to accommodate said flange.

15. Delivery system according to one of the preceding claims, wherein contact surfaces of said container bottom and said closure element are complementarily shaped, and at least one of said contact surfaces comprises a blind hole for accommodating a tip of said hollow needle.

16. Delivery system according to claim 15, wherein said contact surfaces are generally flat-shaped or ellipsoidically or spherically shaped.

17. Delivery system according to one of the preceding claims, wherein said guiding groove extends along and/or around said longitudinal axis.

18. Delivery system according to one of the preceding claims, wherein said guiding groove is such that a relative movement of said extraction unit with respect to said container unit is carried out by (i) rotating and/or advancing said extraction unit along said longitudinal axis to longitudinally move said hollow needle by a first distance (h2) to make said hollow needle penetrate said closure element, and (ii) further rotating and/or retracting said extraction unit to longitudinally move said hollow needle by a second distance (h2 - h1) to retract said hollow needle to a complete extraction allowing position.

19. Delivery system according to claim 18, wherein said guiding groove is such that said relative movement of said extraction unit with respect to said container unit is further carried out by (iii) rotating said extraction unit to a locking position, preferably without longitudinally moving said hollow needle.

20. Delivery system according to one of the preceding claims, wherein said delivery system is a modular system comprising said extraction unit as a first module and said container unit as a second module.

21. Delivery system according to claim 20, wherein said second module comprises said container, said attachment element, and said closure element as a first, a second, and a third sub-module, respectively.

22. Delivery system according to claim 20 or 21, wherein at least one of said modules is sterilizable.

23. Delivery system according to one of the preceding claims, wherein said extraction unit is elastically biased in a direction away from said container unit.

24. Delivery system according to claim 23, further comprising an elastic element arranged between said extraction unit and said container unit.

## Patentansprüche

1. Abgabesystem (10) zum Abgeben von medizinischen oder pharmazeutischen Verbindungen, mit:
- einer Behältereinheit (2000), die umfasst:
- einen Behälter (2100) zum Speichern der Verbindungen, wobei der Behälter eine Umfangswand (2102), ein erstes Ende, das durch einen Behälterboden (2104) verschlossen ist, und ein offenes zweites Ende (1106) umfasst, und wobei die Behältereinheit (2000) eine Längsachse besitzt, und
- ein Verschlusselement (2200), das in dem Behälter (2100) in einem fluiddichten Kontakt mit der Umfangswand aufgenommen ist, und
- einer Extraktionseinheit (1000), die ein Durchstech- und Abführungsmittel mit einer Hohlnadel (1102), die dazu geeignet ist, das Verschlusselement (2200) zu durchstechen,
wobei:
- die Extraktionseinheit (1000) auf den Behälter (2100) aufgepasst ist, um entlang der Längsachse relativ zu dem Behälter (2100) in Richtung des Behälterbodens (2104) bewegbar zu sein;
wobei das Abgabesystem **dadurch gekennzeichnet ist, dass**:
- die Extraktionseinheit einen erste Kulissenbewegungsabschnitt (1107) umfasst und der Behälter (2100) einen zweiten Kulissenbewegungsabschnitt (2305) umfasst, und
- einer von dem erste Kulissenbewegungsabschnitt und dem zweiten Kulissenbewegungsabschnitt einen Vorsprung (1106) umfasst und der jeweils weitere eine Führungsnut (2304) umfasst, wobei der Vorsprung und die Führungsnut in Eingriff gebracht werden können, um so eine Kulissenbewegung zu erzeugen, die dazu geeignet ist, die Extraktionseinheit (1000) relativ zu dem Behälter (2100) auf einem vorbestimmten Weg in Richtung des Behälterbodens (2104) zu bewegen, so dass die Hohlnadel (1102) das Verschlusselement (2200) durchsticht.

2. Abgabesystem nach Anspruch 1, wobei der erste Kulissenbewegungsabschnitt an dem Durchstech- und Abführungsmittel angeordnet ist.

3. Abgabesystem nach Anspruch 1 oder 2, wobei der erste Kulissenbewegungsabschnitt in den zweiten Kulissenbewegungsabschnitt eingeführt ist.

4. Abgabesystem nach einem der vorherigen Ansprüche, wobei der zweite Kulissenbewegungsabschnitt in einem Befestigungselement angeordnet ist, das an einem Randabschnitt des Behälters befestigt ist, wobei der Randabschnitt das offene zweite Ende bildet.

5. Abgabesystem nach Anspruch 4, wobei sich das Durchstech- und Abführungsmittel durch ein Durchgangsloch des Befestigungselements erstreckt, wobei das Durchstech- und Abführungsmittel und das Durchgangsloch radial so bemessen sind, dass sie eine Spielpassung bilden.

6. Abgabesystem nach Anspruch 5, wobei das Befestigungselement einen ersten Überlappungsabschnitt umfasst, der den Behälter nach außen überlappt.

7. Abgabesystem nach Anspruch 5 oder Anspruch 6, wobei das Befestigungselement einen zweiten Überlappungsabschnitt umfasst, der den Behälter nach innen überlappt.

8. Abgabesystem nach Anspruch 6 oder 7, wobei wenigstens der erste Überlappungsabschnitt aus einem elastischen Material gebildet ist.

9. Abgabesystem nach einem der Ansprüche 4 bis 8, wobei ein Abschnitt des Befestigungselements, der den zweiten Kulissenbewegungsabschnitt bildet, aus einem nicht-elastischen Material gebildet ist.

10. Abgabesystem nach einem der vorherigen Ansprüche, wobei die Extraktionseinheit einen Griffabschnitt umfasst, der mit dem Durchstech- und Abführungsmittel verbunden ist.

11. Abgabesystem nach einem der vorherigen Ansprüche, wobei die Extraktionseinheit ein Abgabe-Ende umfasst, das als Teil eines Luer-Lock-Verbinders ausgebildet ist.

12. Abgabesystem nach einem der Ansprüche 1 bis 10, wobei die Extraktionseinheit ein Abgabe-Ende umfasst, das als Sprühventil ausgebildet ist.

13. Abgabesystem nach einem der Ansprüche 1 bis 10, wobei die Extraktionseinheit ein Abgabe-Ende umfasst, das als Kanüle ausgebildet ist.

14. Abgabesystem nach einem der Ansprüche 6 bis o 13, wobei der Behälter einen Flansch umfasst, der sich entlang des zweiten offenen Endes erstreckt, und der erste Überlappungsabschnitt eine kreisförmige Nut umfasst, die dazu geeignet ist, den Flansch aufzunehmen.

15. Abgabesystem nach einem der vorherigen Ansprüche, wobei Kontaktoberflächen des Behälterbodens und des Verschlusselements komplementär geformt sind und wenigstens eine der Kontaktoberflächen ein Blindloch zur Aufnahme einer Spitze der Hohlnadel umfasst.

16. Abgabesystem nach Anspruch 15, wobei die Kontaktoberflächen allgemein flach oder ellipsoid oder sphärisch geformt sind.

17. Abgabesystem nach einem der vorherigen Ansprüche, wobei sich die Führungsnut entlang und/oder um die Längsachse erstreckt.

18. Abgabesystem nach einem der vorherigen Ansprüche, wobei die Führungsnut derart ist, dass eine Relativbewegung der Extraktionseinheit bezüglich der Behältereinheit durch (i) Drehen und/oder Vorwärtsbewegen der Extraktionseinheit entlang der Längsachse zum Längsbewegen der Hohlnadel um eine erste Distanz (h2), so dass die Hohlnadel das Verschlusselement durchsticht, und (ii) Weiterdrehen und/oder Zurückziehen der Extraktionseinheit zum Längsbewegen der Hohlnadel um eine zweite Distanz (h2 - h1), so dass die Hohlnadel zu einer Position, die ein vollständiges Extrahieren erlaubt, zurückgezogen wird, durchgeführt wird.

19. Abgabesystem nach Anspruch 18, wobei die Führungsnut derart ist, dass die Relativbewegung der Extraktionseinheit bezüglich der Behältereinheit durch (iii) Drehen der Extraktionseinheit zu einer Verriegelungsposition, vorzugsweise ohne Längsbewegung der Hohlnadel, fortgesetzt wird.

20. Abgabesystem nach einem der vorherigen Ansprüche, wobei das Abgabesystem ein modulares System ist, das die Extraktionseinheit als ein erstes Modul und die Behältereinheit als ein zweites Modul umfasst.

21. Abgabesystem nach Anspruch 20, wobei das zweite Modul den Behälter, das Befestigungselement und das Verschlusselement als ein erstes, ein zweites bzw. ein drittes Teilmodul umfasst.

22. Abgabesystem nach Anspruch 20 oder 21, wobei wenigstens eines der Module sterilisiert ist.

23. Abgabesystem nach einem der vorherigen Ansprüche, wobei die Extraktionseinheit in eine Richtung von der Behältereinheit weg elastisch vorgespannt ist.

24. Abgabesystem nach Anspruch 23, das ferner ein elastisches Element umfasst, das zwischen der Extraktionseinheit und der Behältereinheit angeordnet ist.

## Revendications

1. Système d'administration pour l'administration de composés médicaux ou pharmaceutiques, comprenant :
- une unité de récipient (2000), comprenant :
- un récipient (2100) pour stocker lesdits composés, dans lequel ledit récipient a une paroi circonférentielle (2102), une première extrémité fermée par un fond de récipient (2104) et une seconde extrémité ouverte (2106), et dans lequel ladite unité de récipient (2000) a un axe longitudinal, et
- un élément de fermeture (2200) logé à l'intérieur dudit récipient (2100) dans un contact étanche aux fluides avec ladite paroi circonférentielle, et
- une unité d'extraction (1000) comprenant un moyen de pénétration et d'évacuation ayant une aiguille creuse (1102) conçue pour pénétrer dans ledit élément de fermeture (2200),
dans lequel :
- ladite unité d'extraction (1000) est montée sur ledit récipient (2100) de façon à être mobile par rapport audit récipient (2100) le long de l'axe longitudinal (A) vers le fond de récipient (2104),
le système d'administration étant **caractérisé en ce que** :
ladite unité d'extraction comprend une première partie de mouvement de liaison (1107), et ledit récipient (2100) comprend une seconde partie de mouvement de liaison (2305), et
- l'une de ladite première partie de mouvement de liaison et de ladite seconde partie de mouvement de liaison comprend une saillie (1106) et l'autre partie respective comprend une rainure de guidage (2304),
ladite saillie et ladite rainure de guidage pouvant venir en prise l'une avec l'autre pour former un mouvement de liaison adapté à amener ladite unité d'extraction (1000) à se déplacer par rapport au récipient (2100) d'une manière prédéterminée vers ledit fond de récipient (2104) pour ainsi amener ladite aiguille creuse (1102) à pénétrer dans ledit élément de fermeture (2200).

2. Système d'administration selon la revendication 1, dans lequel ladite première partie de mouvement de liaison est agencée au niveau dudit moyen de pénétration et d'évacuation.

3. Système d'administration selon la revendication 1 ou 2, dans lequel ladite première partie de mouvement de liaison est insérée dans ladite seconde partie de mouvement de liaison.

4. Système d'administration selon l'une des revendications précédentes, dans lequel ladite seconde partie de mouvement de liaison est agencée en un élément de fixation fixé à une partie rebord dudit récipient, ladite partie rebord formant la seconde extrémité ouverte.

5. Système d'administration selon la revendication 4, dans lequel ledit moyen de pénétration et d'évacuation s'étend à travers un trou traversant dudit élément de fixation, dans lequel ledit moyen de pénétration et d'évacuation et ledit trou traversant sont radialement dimensionnés pour former un ajustement lâche.

6. Système d'administration selon la revendication 5, dans lequel ledit élément de fixation a une première partie chevauchante chevauchant vers l'extérieur ledit récipient.

7. Système d'administration selon la revendication 5 ou la revendication 6, dans lequel ledit élément de fixation a une seconde partie chevauchante chevauchant vers l'intérieur ledit récipient.

8. Système d'administration selon la revendication 6 ou 7, dans lequel au moins ladite première partie chevauchante est en un matériau élastique.

9. Système d'administration selon l'une quelconque des revendications 4 à 8, dans lequel une partie dudit élément de fixation formant ladite seconde partie de mouvement de liaison est en un matériau non élastique.

10. Système d'administration selon l'une des revendications précédentes, dans lequel ladite unité d'extraction comprend une partie de préhension raccordée audit moyen de pénétration et d'évacuation.

11. Système d'administration selon l'une des revendications précédentes, dans lequel ladite unité d'extraction a une extrémité d'administration formée comme une partie d'un raccord Luer lock.

12. Système d'administration selon l'une des revendications 1 à 10, dans lequel ladite unité d'extraction a une extrémité d'administration formée comme une partie d'une vanne de pulvérisation.

13. Système d'administration selon l'une des revendications 1 à 10, dans lequel ladite unité d'extraction a une extrémité d'administration sous la forme d'une canule.

14. Système d'administration selon l'une des revendications 6 à 13, dans lequel ledit récipient a une bride s'étendant le long de ladite seconde extrémité ouverte, et ladite première partie chevauchante a une rainure circulaire adaptée à loger ladite bride.

15. Système d'administration selon l'une des revendications précédentes, dans lequel les surfaces de contact dudit fond de récipient et dudit élément de fermeture ont une forme complémentaire, et au moins l'une desdites surfaces de contact comprend un trou borgne pour loger une pointe de ladite aiguille creuse.

16. Système d'administration selon la revendication 15, dans lequel lesdites surfaces de contact sont généralement de forme plate ou de forme ellipsoïde ou sphérique.

17. Système d'administration selon l'une des revendications précédentes, dans lequel ladite rainure de guidage s'étend le long et/ou autour dudit axe longitudinal.

18. Système d'administration selon l'une des revendications précédentes, dans lequel ladite rainure de guidage est telle qu'un mouvement relatif de ladite unité d'extraction relativement à ladite unité de récipient est réalisé par (i) mise en rotation et/ou progression de ladite unité d'extraction le long dudit axe longitudinal pour déplacer longitudinalement ladite aiguille creuse d'une première distance (h2) pour amener ladite aiguille creuse à pénétrer dans ledit premier élément de fermeture, et (ii) en outre mise en rotation et/ou rétraction de ladite unité d'extraction pour déplacer longitudinalement ladite aiguille creuse d'une seconde distance (h2 - h1) pour rétracter ladite aiguille creuse jusqu'à une position permettant l'extraction complète.

19. Système d'administration selon la revendication 18, dans lequel ladite rainure de guidage est telle que le mouvement relatif de ladite unité d'extraction relativement à ladite unité de récipient est en outre réalisé par (iii) mise en rotation de ladite unité d'extraction jusqu'à une position de verrouillage, de préférence sans déplacer longitudinalement ladite aiguille creuse.

20. Système d'administration selon l'une des revendications précédentes, dans lequel ledit système d'administration est un système modulaire comprenant ladite unité d'extraction en tant que premier module et ladite unité de récipient en tant que second module.

21. Système d'administration selon la revendication 20, dans lequel ledit second module comprend ledit récipient, ledit élément de fixation, et ledit élément de fermeture en tant que premier, deuxième, et troisième sous-modules, respectivement.

22. Système d'administration selon la revendication 20 ou 21, dans lequel au moins l'un desdits modules peut être stérilisé.

23. Système d'administration selon l'une des revendications précédentes, dans lequel ladite unité d'extraction est élastiquement sollicitée dans une direction à l'écart de ladite unité de récipient.

24. Système d'administration selon la revendication 23, comprenant en outre un élément élastique agencé entre ladite unité d'extraction et ladite unité de récipient.
